# EUROPEAN PATENT SPECIFICATION

(11) **EP 4 616 810 B1**
(45) Date of publication and mention of the grant of the patent: **14.10.2026**
(21) Application number: 25163338.4
(22) Date of filing: 12.03.2025
(51) Int. Cl.: A61B 6/12, A61B 6/00, A61B 6/50

(54) **RADIATION IMAGE PROCESSING DEVICE, RADIATION IMAGE PROCESSING METHOD, AND RADIATION IMAGE PROCESSING PROGRAM**
STRAHLUNGSBILDVERARBEITUNGSVORRICHTUNG, STRAHLUNGSBILDVERARBEITUNGSVERFAHREN UND STRAHLUNGSBILDVERARBEITUNGSPROGRAMM
DISPOSITIF DE TRAITEMENT D'IMAGE DE RAYONNEMENT, PROCÉDÉ DE TRAITEMENT D'IMAGE DE RAYONNEMENT ET PROGRAMME DE TRAITEMENT D'IMAGE DE RAYONNEMENT

(30) Priority: 12.03.2024 JP 2024037900
(43) Date of publication of application: 17.09.2025
(73) Proprietor: FUJIFILM Corporation, Tokyo 106-8620 (JP)
(72) Inventor: TAKAHASHI, Tomoyuki, Kaisei-machi (JP)
(74) Representative: Dehns Germany Partnerschaft mbB

(56) References cited:
- US-A1- 2022 167 935
- US-A1- 2022 287 664
- US-A1- 2023 153 970
- US-A1- 2024 016 464
- US-A1- 2024 104 729

## Description

### BACKGROUND

### Technical Field

The present disclosure relates to a radiation image processing device, a radiation image processing method, and a radiation image processing program.

### Related Art

In the related art, energy subtraction processing using two radiation images obtained by irradiating a subject with two types of radiation having different energy distributions by using the fact that an attenuation amount of the transmitted radiation differs in accordance with the substance constituting the subject has been known. The energy subtraction processing is a method in which respective pixels of the two radiation images obtained as described above are associated with each other, and subtraction is performed after multiplying a weight coefficient based on an attenuation coefficient in accordance with a component between pixels to acquire an image in which specific components, such as a bone part and a soft part, included in the radiation image are separated. In addition, a method of separating a soft tissue of the subject into fat and muscle by energy subtraction processing has also been proposed (see JP2023-047911A and JP2022-056084A).

In order to separate a plurality of components included in the subject by the energy subtraction processing, the attenuation coefficient of the soft part is required. The soft part does not consist of a single composition, and a plurality of components, such as fat and muscle, are complicatedly mixed. The components of the soft part vary greatly depending on the individual. Therefore, in a case in which the attenuation coefficient of the soft part is not obtained in accordance with a ratio between the fat and the muscle, it is not possible to accurately separate a plurality of components, such as the bone part and the soft part, in a case in which the processing is performed using the characteristics related to the attenuation of the radiation, such as the energy subtraction processing.

In this case, it is considered to separate the soft part into the fat and the muscle. However, in the energy subtraction processing, since two radiation images obtained by two types of radiation having different energy distributions are used, only radiation images of two components, such as the bone part and the soft part, can be obtained. That is, in a case in which n radiation images obtained by n types of radiation having different energy distributions are used, only n component images can be obtained.

US 2023/0153970 A1 describes generating bone and soft tissue images through energy subtraction and obtaining a total thickness image by adding the derived component thicknesses to enhance the visibility of contrast agents.

### SUMMARY OF THE INVENTION

The present disclosure has been made in view of the above-described circumstances, and an object of the present disclosure is to accurately separate n components of a subject included in a radiation image by using n radiation images obtained by n - 1 (n ≥ 3) types of radiation having different energy distributions.

The present disclosure relates to a radiation image processing device comprising: at least one processor, in which the processor is configured to: acquire first to (n - 1)th (n ≥ 3, n is a natural number) radiation images acquired by imaging a subject, which includes a first component consisting of a plurality of compositions and second to nth components each consisting of a single composition, with n - 1 types of radiation having different energy distributions; derive a characteristic of the first component in at least a region of the subject in the first to (n - 1)th radiation images; acquire a body thickness of the subject; derive thicknesses of the first to nth components by using the body thickness, the characteristic of the first component, and the first to (n - 1)th radiation images; and derive first to nth component images in which the first to nth components are enhanced, respectively, based on the thicknesses of the first to nth components.

It should be noted that, in the radiation image processing device according to the present disclosure, the processor may be configured to acquire the body thickness by deriving the body thickness of the subject based on at least one of the first to (n - 1)th radiation images.

In addition, in the radiation image processing device according to the present disclosure, the processor may be configured to derive an attenuation coefficient of the first component as the characteristic of the first component.

In addition, in the radiation image processing device according to the present disclosure, the processor may be configured to derive the attenuation coefficient of the first component as the characteristic of the first component based on information related to an attenuation coefficient of the radiation of each of the plurality of compositions included in the first component and a thickness of each of the plurality of compositions.

In addition, in the radiation image processing device according to the present disclosure, the processor may be configured to: acquire a first radiation image and a second radiation image that are acquired by imaging the subject with two types of radiation having different energy components; derive the characteristic of the first component in at least a region of the subject in the first radiation image or the second radiation image; derive the body thickness of the subject based on at least one of the first radiation image or the second radiation image; derive thicknesses of the first component, a second component, and a third component of the subject by using the body thickness, the characteristic of the first component, the first radiation image, and the second radiation image; and derive a first component image, a second component image, and a third component image in which the first component, the second component, and the third component are enhanced, respectively, based on the thicknesses of the first component, the second component, and the third component.

In addition, in the radiation image processing device according to the present disclosure, the first component, the second component, and the third component may be a soft part and a bone part of the subject and an artificial object in the subject, respectively.

In addition, in the radiation image processing device according to the present disclosure, the compositions of the first component may be fat and muscle.

The present disclosure relates to a radiation image processing method executed by a computer, the radiation image processing method comprising: acquiring first to (n - 1)th (n ≥ 3) radiation images acquired by imaging a subject, which includes a first component consisting of a plurality of compositions and second to nth components each consisting of a single composition, with n - 1 types of radiation having different energy distributions; deriving a characteristic of the first component in at least a region of the subject in the first to (n - 1)th radiation images; acquiring a body thickness of the subject; deriving thicknesses of the first to nth components by using the body thickness, the characteristic of the first component, and the first to (n - 1)th radiation images; and deriving first to nth component images in which the first to nth components are enhanced, respectively, based on the thicknesses of the first to nth components.

The present disclosure relates to a radiation image processing program causing a computer to execute: a procedure of acquiring first to (n - 1)th (n ≥ 3) radiation images acquired by imaging a subject, which includes a first component consisting of a plurality of compositions and second to nth components each consisting of a single composition, with n - 1 types of radiation having different energy distributions; a procedure of deriving a characteristic of the first component in at least a region of the subject in the first to (n - 1)th radiation images; a procedure of acquiring a body thickness of the subject; a procedure of deriving thicknesses of the first to nth components by using the body thickness, the characteristic of the first component, and the first to (n - 1)th radiation images; and a procedure of deriving first to nth component images in which the first to nth components are enhanced, respectively, based on the thicknesses of the first to nth components.

According to the present disclosure, it is possible to accurately separate the n components of the subject included in the radiation image by using the n radiation images obtained by the n - 1 (n ≥ 3) types of radiation having different energy distributions.

### BRIEF DESCRIPTION OF THE DRAWINGS

Fig. 1 is a schematic block diagram showing a configuration of a radiography system to which a radiation image processing device according to an embodiment of the present disclosure is applied.
Fig. 2 is a diagram showing a schematic configuration of the radiation image processing device according to the present embodiment.
Fig. 3 is a diagram showing a functional configuration of the radiation image processing device according to the present embodiment.
Fig. 4 is a diagram showing first and second radiation images.
Fig. 5 is a diagram schematically showing processing performed in the radiation image processing device.
Fig. 6 is a diagram showing attenuation coefficients of fat and muscle.
Fig. 7 is a diagram showing an attenuation amount in accordance with a thickness of the muscle and a thickness of the fat in a high-energy image and a low-energy image.
Fig. 8 is a diagram showing a display screen.
Fig. 9 is a flowchart showing the processing performed in the present embodiment.

### DETAILED DESCRIPTION

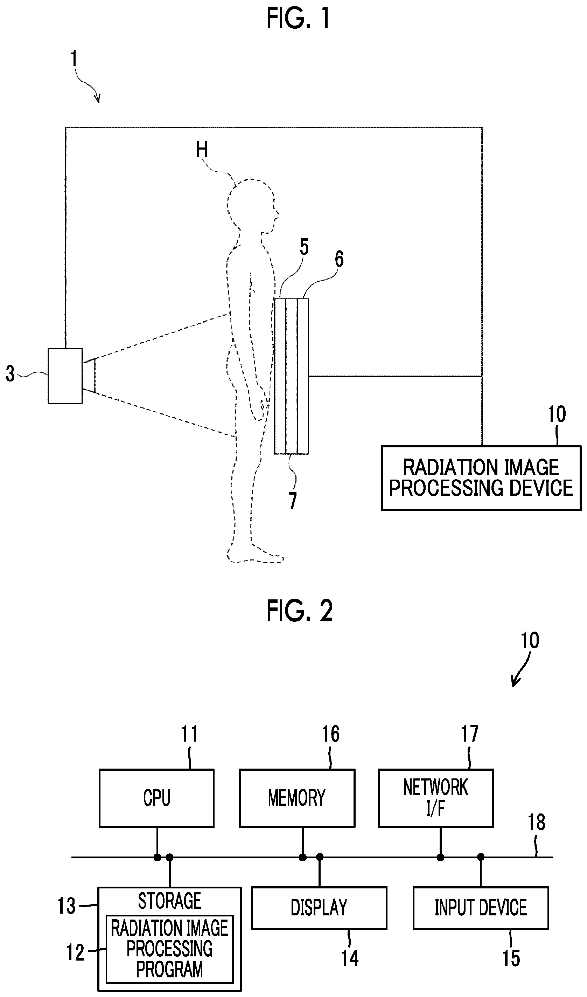

In the following description, an embodiment of the present disclosure will be described with reference to the drawings. Fig. 1 is a schematic block diagram showing a configuration of a radiography system to which a radiation image processing device according to the embodiment of the present disclosure is applied. As shown in Fig. 1, the radiography system according to the present embodiment comprises an imaging apparatus 1 and a radiation image processing device 10 according to the present embodiment.

The imaging apparatus 1 is an imaging apparatus for performing energy subtraction imaging by a so-called one-shot method for changing energy of each of radiation, such as X-rays, emitted from a radiation source 3 and transmitted through a subject H and irradiating a first radiation detector 5 and a second radiation detector 6 with the converted radiation. During the imaging, as shown in Fig. 1, the first radiation detector 5, a radiation energy conversion filter 7 made of a copper plate or the like, and the second radiation detector 6 are disposed in order from a side closest to the radiation source 3, and the radiation source 3 is driven. It should be noted that the first and second radiation detectors 5 and 6 are closely attached to the radiation energy conversion filter 7.

As a result, in the first radiation detector 5, a first radiation image G1 of the subject H by low-energy radiation also including so-called soft rays is acquired. In addition, in the second radiation detector 6, a second radiation image G2 of the subject H by high-energy radiation from which the soft rays are removed is acquired. The first and second radiation images G1 and G2 are input to the radiation image processing device 10.

The first and second radiation detectors 5 and 6 can perform recording and reading-out of the radiation image repeatedly, and a so-called direct-type radiation detector that directly receives emission of the radiation and generates an electric charge may be used, or a so-called indirect-type radiation detector that converts the radiation into visible light and then converts the visible light into an electric charge signal may be used. In addition, as a method for reading out a radiation image signal, it is desirable to use a so-called thin film transistor (TFT) readout method in which the radiation image signal is read out by turning a TFT switch on and off, or a so-called optical readout method in which the radiation image signal is read out by emission of read out light, but other methods may also be used without being limited to these methods.

Hereinafter, the radiation image processing device according to the present embodiment will be described. First, a hardware configuration of the radiation image processing device according to the present embodiment will be described with reference to Fig. 2. As shown in Fig. 2, the radiation image processing device 10 is a computer, such as a workstation, a server computer, and a personal computer, and comprises a central processing unit (CPU) 11, a nonvolatile storage 13, and a memory 16 as a transitory storage region.

In addition, the radiation image processing device 10 comprises a display 14, such as a liquid crystal display, an input device 15, such as a keyboard and a mouse, and a network interface (I/F) 17 connected to a network (not shown). The CPU 11, the storage 13, the display 14, the input device 15, the memory 16, and the network I/F 17 are connected to a bus 18. It should be noted that the CPU 11 is an example of a processor according to the present disclosure.

The storage 13 is implemented by a hard disk drive (HDD), a solid state drive (SSD), a flash memory, and the like. A radiation image processing program 12 installed in the radiation image processing device 10 is stored in the storage 13 as a storage medium. The CPU 11 reads out the radiation image processing program 12 from the storage 13, loads the readout radiation image processing program 12 to the memory 16, and executes the loaded radiation image processing program 12.

The radiation image processing program 12 is stored in a storage device of the server computer connected to the network or in a network storage in a state of being accessible from the outside, and is downloaded and installed in the computer that configures the radiation image processing device 10 in response to the request. Alternatively, the radiation image processing program 12 is distributed in a state of being recorded on a recording medium, such as a digital versatile disc (DVD) or a compact disc read only memory (CD-ROM), and is installed in the computer that configures the radiation image processing device 10 from the recording medium.

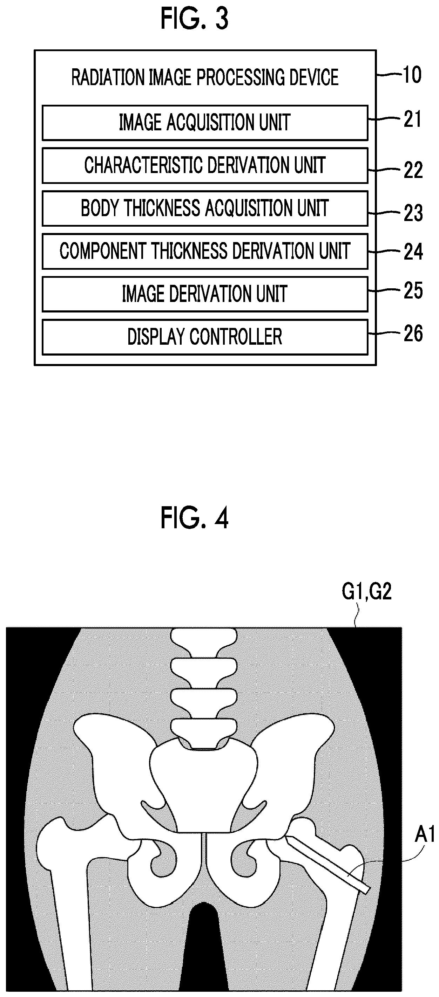

Next, a functional configuration of the radiation image processing device according to the present embodiment will be described. Fig. 3 is a diagram showing the functional configuration of the radiation image processing device according to the present embodiment. As shown in Fig. 3, the radiation image processing device 10 comprises an image acquisition unit 21, a characteristic derivation unit 22, a body thickness acquisition unit 23, a component thickness derivation unit 24, an image derivation unit 25, and a display controller 26. Then, the CPU 11 executes the radiation image processing program 12 to function as the image acquisition unit 21, the characteristic derivation unit 22, the body thickness acquisition unit 23, the component thickness derivation unit 24, the image derivation unit 25, and the display controller 26.

The image acquisition unit 21 acquires the first radiation image G1 and the second radiation image G2 of the subject H from the first and second radiation detectors 5 and 6 by causing the imaging apparatus 1 to perform the energy subtraction imaging of the subject H. In this case, imaging conditions, such as an imaging dose, an energy distribution, a tube voltage, and an SID, are set. The imaging conditions need only be set by input from the input device 15 by a user. The set imaging conditions are stored in the storage 13. It should be noted that the first and second radiation images G1 and G2 may be acquired by a program different from the radiation image processing program according to the first embodiment. In this case, the image acquisition unit 21 reads out the first and second radiation images G1 and G2 stored in the storage 13 from the storage 13 for processing.

Fig. 4 is a diagram showing the first and second radiation images. As shown in Fig. 4, a region of the subject H and a direct radiation region obtained by directly irradiating the radiation detectors 5 and 6 with the radiation are included in the first and second radiation images G1 and G2. A soft region and a bone region are included in the region of the subject H. In addition, in Fig. 4, a region A1 of a screw for fixing a fracture portion of a femur is included in the femur. A soft part component of a human body includes muscle, fat, blood, and water. In the present embodiment, a non-fat tissue including blood and water is treated as the muscle. The screw is an example of an artificial object according to the present disclosure.

The artificial object is an object that is not originally present in the subject H, such as a stent disposed in a blood vessel, silicone disposed in a breast, and a contrast agent, in addition to the screw for fixing the fracture portion as shown in Fig. 4. The soft part component, the bone part component, and the artificial object component of the subject H are examples of a first component, a second component, and a third component according to the present disclosure, respectively. The muscle and the fat are examples of a plurality of compositions according to the present disclosure.

The soft regions of the first and second radiation images G1 and G2 include only the soft part component of the subject H. The bone region and the artificial object region in the first and second radiation images G1 and G2 are actually regions in which the bone part component and the artificial object component are mixed with the soft part component. The soft region is an example of a first component region including only the first component according to the present disclosure, and the bone region is an example of a second component region including the second component according to the present disclosure. The artificial object region is an example of a third component region including the third component according to the present disclosure.

The characteristic derivation unit 22 derives the characteristics of the soft part component in at least a region of the subject H in the first radiation image G1 and the second radiation image G2. In the present embodiment, the characteristic derivation unit 22 derives a soft part attenuation coefficient, which is an attenuation coefficient by the soft part components of low-energy radiation and high-energy radiation, as the characteristics of the first component.

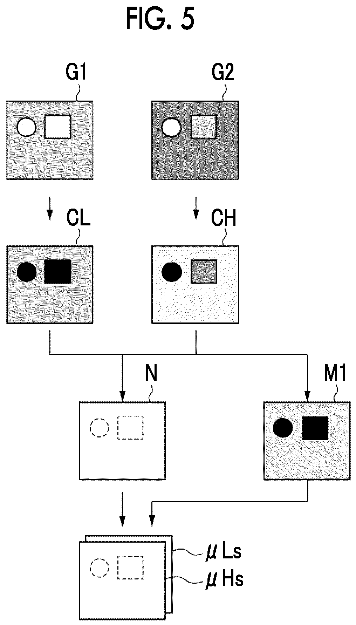

Fig. 5 is a diagram schematically showing processing performed by the characteristic derivation unit 22 in the present embodiment. It should be noted that, in Fig. 5, for simplicity of description, the first radiation image G1 and the second radiation image G2 do not include the direct radiation region, and the soft region includes a rectangular bone region and a circular artificial object region.

In the present embodiment, the characteristic derivation unit 22 first specifies the soft region, the bone region, and the artificial object region in the first radiation image G1 or the second radiation image G2. For this purpose, the characteristic derivation unit 22 derives attenuation characteristics related to the attenuation of the radiation in at least the region of the subject H in the first radiation image G1 or the second radiation image G2, and specifies the soft region, the bone region, and the artificial object region based on the attenuation characteristics in the region of the subject H. In the present embodiment, the characteristic derivation unit 22 derives a first attenuation image CL and a second attenuation image CH, which represent the attenuation amounts of the radiation due to the subject H, from the first radiation image G1 and the second radiation image G2, respectively, and derives an attenuation ratio, which is a ratio between corresponding pixels of the first attenuation image CL and the second attenuation image CH, as the attenuation characteristics.

A pixel value of the first attenuation image CL represents the attenuation amount of the low-energy radiation due to the subject H, and a pixel value of the second attenuation image CH represents the attenuation amount of the high-energy radiation due to the subject H. The first attenuation image CL and the second attenuation image CH are derived from the first radiation image G1 and the second radiation image G2 by Expression (1) and Expression (2). In the expression (1), Gd1 is the pixel value of the direct radiation region in the first radiation image G1, and, in the expression (2), Gd2 is the pixel value of the direct radiation region in the second radiation image G2. $CL \left(x, y\right) = Gd 1 - G 1 \left(x, y\right)$ (1) $CH \left(x, y\right) = Gd 2 - G 2 \left(x, y\right)$ (2)

Next, the characteristic derivation unit 22 derives an attenuation ratio map representing the attenuation ratio of radiation between the first radiation image G1 and the second radiation image G2. Specifically, an attenuation ratio map M1 is derived by deriving a ratio between the corresponding pixels of the first attenuation image CL and the second attenuation image CH by Expression (3). The attenuation ratio is an example of a characteristic related to attenuation of the radiation according to the present disclosure. $M 1 \left(x, y\right) = CL \left(x, y\right) / CH \left(x, y\right)$ (3)

Here, in the first radiation image G1 and the second radiation image G2, the attenuation ratio of the region including only the soft part component is less than the attenuation ratio of the region including the bone part component and the artificial object component. Therefore, the characteristic derivation unit 22 compares the attenuation ratios of the pixels of the attenuation ratio map M1, specifies a region consisting of pixels in which the attenuation ratio is greater than a predetermined threshold value as the bone region and the artificial object region, and specifies a region in which the attenuation ratio is less than the threshold value as the soft region.

Further, in the present embodiment, the characteristic derivation unit 22 derives the soft part attenuation coefficient µLs for the low-energy radiation and the soft part attenuation coefficient µHs for the high-energy radiation by using the first attenuation image CL and the second attenuation image CH. Hereinafter, the derivation of the soft part attenuation coefficients µLs and µHs will be described.

In the present embodiment, the soft part attenuation coefficient is derived on the assumption that, among the compositions constituting the soft part, the composition having the highest density is the muscle, the composition having a lower density is the fat, and a mixed composition in which the fat and the muscle are mixed has an intermediate value of both attenuation coefficients. First, the characteristic derivation unit 22 calculates provisional soft part attenuation coefficients µ0Ls and µ0Hs for each of the low-energy radiation and the high-energy radiation by setting the ratio of the fat at each pixel position to N% and performing weighting addition of the attenuation coefficient of the fat and the attenuation coefficient of the muscle at a ratio of N:100 - N while sequentially increasing N from zero.

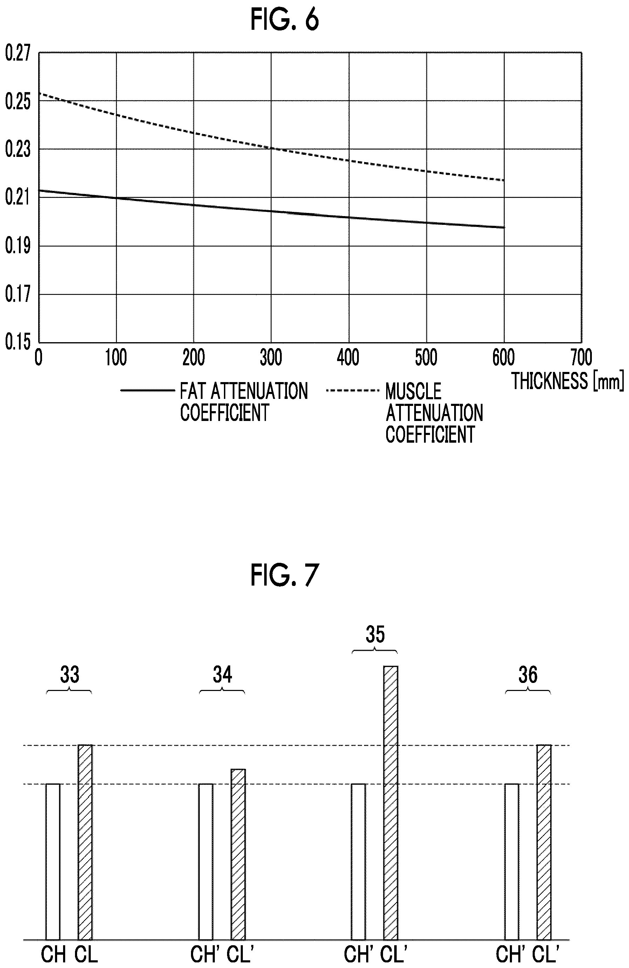

It should be noted that, in a case in which the fat and the muscle overlap each other, the attenuation coefficient is changed due to the influence of the radiation quality hardening of the component (usually the fat) present on the radiation source 3 side, but, in the present embodiment, the influence of the radiation quality hardening is not taken into consideration. Therefore, N%, which is the ratio of the fat used in the present embodiment, does not match the actual body fat percentage of the subject H. The processing is based on the assumption that the actual soft part attenuation coefficient is a value between the attenuation coefficient of the fat and the attenuation coefficient of the muscle shown in Fig. 6. It should be noted that the horizontal axis of Fig. 6 represents the thickness (mm) of the fat and the muscle.

Next, the characteristic derivation unit 22 calculates a body thickness TN in a case in which the ratio of the fat is N% by Expression (4) from the pixel value of the first attenuation image CL and the provisional soft part attenuation coefficient µ0Ls for the low-energy image. In this case, the body thickness TN is calculated on the assumption that the pixels, which include the bone part and the artificial object, are also composed of only the soft part. $TN \left(x, y\right) = CL \left(x, y\right) / μ 0 Ls \left(x, y\right)$ (4)

Next, the characteristic derivation unit 22 calculates an attenuation amount CHN1 of the high-energy radiation according to Expression (5) from the body thickness TN calculated by Expression (4) and the provisional soft part attenuation coefficient µ0Hs for the high-energy radiation. Then, the second attenuation image CH is subtracted from the attenuation amount CHN1 by Expression (6) to calculate a difference value ΔCH. $CHN 1 \left(x, y\right) = TN \left(x, y\right) \times μ 0 Hs \left(x, y\right)$ (5) $ΔCH \left(x, y\right) = CHN 1 \left(x, y\right) - CH \left(x, y\right)$ (6)

A case in which the difference value ΔCH is a negative value means that the provisional soft part attenuation coefficients µ0Ls and µ0Hs are less than a correct answer soft part attenuation coefficient, that is, closer to the fat. A case in which the difference value ΔCH is a positive value means that the provisional soft part attenuation coefficients µ0Ls and µ0Hs are closer to the muscle. The characteristic derivation unit 22 calculates the provisional soft part attenuation coefficients µ0Ls and µ0Hs for all the pixels of the first attenuation image CL and the second attenuation image CH while changing N such that the difference value ΔCH approaches zero. Then, N in a case in which the difference value ΔCH is zero or equal to or less than a predetermined threshold value is determined as the ratio of the fat for the pixel. In addition, the characteristic derivation unit 22 determines the provisional soft part attenuation coefficients µ0Ls and µ0Hs in a case of calculating the determined ratio N of the fat, as the soft part attenuation coefficients µLs and µHs in a case in which the thickness of the soft part is the body thickness TN. It should be noted that the change need only be made such that the ratio N of the fat is increased in a case in which the difference value ΔCH is a negative value, and the ratio N of the fat is decreased in a case in which the difference value ΔCH is a positive value.

In the present embodiment, the characteristic derivation unit 22 derives the soft part attenuation coefficients µLs and µHs as the characteristics of the first component in the soft region. On the other hand, the characteristic derivation unit 22 derives the soft part attenuation coefficients µLs and µHs in the bone region and the artificial object region by interpolating the soft part attenuation coefficient of the soft region within a range of a predetermined distance from the periphery of the bone region and the artificial object region, for example, from a boundary between the bone region and the artificial object region. It should be noted that, instead of the interpolation, a median value of the soft part attenuation coefficients µLs and µHs in the soft region, an average value thereof, or a value thereof that is a predetermined ratio from the small attenuation coefficient side may be derived as the soft part attenuation coefficients µLs and µHs for the bone region and the artificial object region.

The body thickness acquisition unit 23 acquires the body thickness of the subject H by deriving the body thickness of the subject H for each pixel of the first and second radiation images G1 and G2 based on at least one of the first radiation image G1 or the second radiation image G2. Here, the body thickness is a thickness in a direction in which the radiation is transmitted through the subject H, that is, a thickness in a direction perpendicular to the first and second radiation images G1 and G2. Since the body thickness is derived for each pixel of the first and second radiation images G1 and G2, the body thickness acquisition unit 23 derives a body thickness distribution in at least one of the first radiation image G1 or the second radiation image G2. In the derivation of the body thickness, the body thickness acquisition unit 23 uses the first radiation image G1 acquired by the radiation detector 5 on the side close to the subject H. It should be noted that the second radiation image G2 may be used. In addition, regardless of which image is used, a low-frequency image representing a low-frequency component of the image may be derived, to use the low-frequency image to derive the body thickness.

In the present embodiment, the body thickness acquisition unit 23 derives the body thickness of the subject H by assuming that a brightness distribution of the first radiation image G1 coincides with the body thickness distribution of the subject H, and converting the pixel value of the first radiation image G1 into the thickness by using an attenuation coefficient of the soft part of the subject H. Instead of the above, the body thickness acquisition unit 23 may measure the thickness of the subject H by using a sensor or the like. Also, the body thickness acquisition unit 23 may derive the body thickness by approximating the body thickness of the subject H with a model such as a cube or an elliptical column. The body thickness acquisition unit 23 may derive the body thickness of the subject H by any method such as a method described in JP2015-043959A.

The component thickness derivation unit 24 derives the thicknesses of the first component to the third component included in the subject H, that is, the thickness of the soft part, the thickness of the bone part, and the thickness of the artificial object, by using the body thickness derived by the body thickness acquisition unit 23, the characteristics of the first component (that is, the soft part attenuation coefficients µLs and µHs) derived by the characteristic derivation unit 22, the first radiation image G1, and the second radiation image G2.

Here, the attenuation amount of the radiation by the subject H is determined depending on the thickness of the soft part, the thickness of the bone part, the thickness of the artificial object, and the radiation quality (high-energy or low-energy). Therefore, in a case in which the attenuation coefficient representing an attenuation rate per unit thickness is µ, attenuation amounts CL0 and CH0 of the radiation at each pixel position in each of the low-energy image and the high-energy image can be represented by Expression (7) and Expression (8). In Expression (7) and Expression (8), ts is the thickness of the soft part, tb is the thickness of the bone part, ta is the thickness of the artificial object, µLs is the soft part attenuation coefficient of the low-energy radiation, µLb is the bone part attenuation coefficient of the low-energy radiation, µLa is the artificial object attenuation coefficient of the low-energy radiation, µHs is the soft part attenuation coefficient of the high-energy radiation, µHb is the bone part attenuation coefficient of the high-energy radiation, and µHa is the artificial object attenuation coefficient of the high-energy radiation. The attenuation coefficient represents an attenuation rate of radiation per unit thickness. $CL 0 = μLs \left(ts, tb, ta\right) \times ts + μLb \left(ts, tb, ta\right) \times tb + μLa \left(ts, tb, ta\right) \times ta$ (7) $CH 0 = μHs \left(ts, tb, ta\right) \times ts + μHb \left(ts, tb, ta\right) \times tb + μHa \left(ts, tb, ta\right) \times ta$ (8)

In Expression (7) and Expression (8), the attenuation amount CL0 of the low-energy image corresponds to the pixel value of the first attenuation image CL, and the attenuation amount CH0 of the high-energy image corresponds to the pixel value of the second attenuation image CH. It should be noted that Expression (7) and Expression (8) represent a relationship between the first attenuation image CL and the second attenuation image CH in each pixel, but (x,y) representing the pixel position is omitted. In Expression (9) to Expression (13) shown later, (x,y) representing the pixel position is also omitted.

In Expression (7) and Expression (8), the variables are the thickness ts of the soft part, the thickness tb of the bone part, and the thickness ta of the artificial object. Since there are three variables, the thickness ts of the soft part, the thickness tb of the bone part, and the thickness ta of the artificial object cannot be derived only by two Expressions (7) and (8). In the present embodiment, the body thickness acquisition unit 23 acquires the body thickness T of the subject H. A relationship between the body thickness T, the thickness ts of the soft part, the thickness tb of the bone part, and the thickness ta of the artificial object is expressed by Expression (9). Expression (9) is modified for the thickness ta of the artificial object, to obtain Expression (10). $T = ts + tb + ta$ (9) $ta = T - ts - tb$ (10)

Since the attenuation amount CL0 of the low-energy image corresponds to the pixel value of the first attenuation image CL and the attenuation amount CH0 of the high-energy image corresponds to the pixel value of the second attenuation image CH, in a case in which Expression (10) is substituted into Expression (7) and Expression (8), Expression (11) and Expression (12) are obtained. 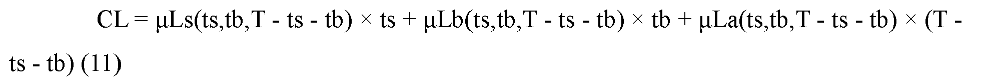 CL = µLs(ts,tb, T - ts - tb) × ts + µLb(ts,tb,T - ts - tb) × tb + µLa(ts,tb,T - ts - tb) × (T - ts - tb) (11) 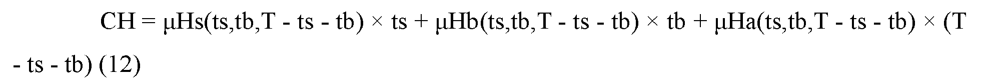 CH = µHs(ts,tb,T - ts - tb) × ts + µHb(ts,tb, T - ts - tb) × tb + µHa(ts,tb,T - ts - tb) × (T - ts - tb) (12)

In the present embodiment, since the body thickness T is acquired by the body thickness acquisition unit 23, the variables in Expression (11) and Expression (12) are two variables of the thickness ts of the soft part and the thickness tb of the bone part. Therefore, by solving Expression (11) and Expression (12) with the thickness ts of the soft part and the thickness tb of the bone part as variables, the thickness ts of the soft part and the thickness tb of the bone part can be derived. Further, the thickness ta of the artificial object can be derived by Expression (10) from the body thickness T, the derived thickness ts of the soft part, and the derived thickness tb of the bone part.

For solving Expression (11) and Expression (12), the soft part attenuation coefficients µLs and µHs, the bone part attenuation coefficients µLb and µHb, and the artificial object attenuation coefficients µLa and µHa are required for each of the low-energy radiation and the high-energy radiation.

The soft part attenuation coefficients µLs and µHs derived by the characteristic derivation unit 22 are used. On the other hand, since there is no difference in composition between the bone part and the artificial object depending on the subject H, the bone part attenuation coefficients µLb and µHb and the artificial object attenuation coefficients µLa and µHa in accordance with the thickness ts of the soft part, the thickness tb of the bone part, and the thickness ta of the artificial object can be prepared in advance.

Here, in a procedure of the radiation transmitted through the subject H, a low-energy component of the radiation is absorbed by the subject H, and beam hardening occurs in which the radiation energy is increased. Therefore, in the present embodiment, the bone part attenuation coefficients µLb and µHb and the artificial object attenuation coefficients µLa and µHa are derived in advance in consideration of the beam hardening, and then stored in the storage 13.

In a case in which the radiation is transmitted through the subject including the artificial object, inside the human body, the soft part, the bone part, and the artificial object are transmitted in a complicated order in accordance with the overlap thereof. On the other hand, in a case in which monochromatic radiation is considered, the monochromatic radiation has no change in spectrum, so that the soft part, the bone part, and the artificial object have a constant attenuation coefficient. In a case in which the total thicknesses of the soft part, the bone part, and the artificial object are the same, the radiation dose after the transmission through the subject is always the same regardless of the order in which the radiation is transmitted through the respective components in the subject.

Radiation actually emitted to the subject is continuous radiation having a wide spectrum, but is a collection of the monochromatic radiation. Therefore, even in a case of the continuous radiation, in a case in which the total thicknesses of the soft part, the bone part, and the artificial object are the same, the radiation dose after the radiation is transmitted through the subject is always the same regardless of the order in which the components are transmitted.

In the present embodiment, the thickness ts of the soft part, the thickness tb of the bone part, and the thickness ta of the artificial object are required to derive a component image described below, and the information on how the soft part, the bone part, and the artificial object overlap each other is not required. Therefore, in the present embodiment, the attenuation coefficient is derived by using three types of models, that is, a model consisting of only the soft part, a simple model in which the radiation is first transmitted through the soft part and then transmitted through the bone part, and a simple model in which the radiation is first transmitted through the soft part, then transmitted through the bone part, and then transmitted through the artificial object. It should be noted that, in the model, the soft part, the bone part, and the artificial object are objects having attenuation coefficients corresponding to the soft part, the bone part, and the artificial object, respectively. The order of the components in the model is not limited to the soft part, the bone part, and the artificial object, and any order can be used.

As the soft part attenuation coefficients µLs and µHs, the values derived by the characteristic derivation unit 22 as described above are used. Therefore, the model consisting of only the soft part is not used.

For the bone part attenuation coefficient, a model consisting of the soft part and the bone part, that is, a model consisting of an object having the attenuation coefficient corresponding to each of the soft part and the bone part is used, and the low-energy radiation and the high-energy radiation are respectively emitted to the model while changing the thicknesses of the soft part and the bone part, to derive the low-energy image and the high-energy image. Then, a ratio of the pixel value of each pixel of the low-energy image and the high-energy image to an image (hereinafter, referred to as a direct image) acquired by the low-energy radiation and the high-energy radiation in a case in which the soft part and the bone part are not present is derived, and the ratio is further divided by the thickness of the bone part to derive the bone part attenuation coefficients µLb and µHb for various thicknesses of the bone part. In derivation of the bone part attenuation coefficients µLb and µHb, the thickness of the soft part is also taken into consideration, so that the bone part attenuation coefficients µLb and µHb are obtained in consideration of the beam hardening of the soft part.

For the artificial object attenuation coefficient, a model consisting of the soft part, the bone part, and the artificial object, that is, a model consisting of an object having the attenuation coefficient corresponding to each of the soft part, the bone part, and the artificial object is used, and the low-energy radiation and the high-energy radiation are respectively emitted to the model while changing the thicknesses of the soft part, the bone part, and the artificial object, to derive the low-energy image and the high-energy image. A ratio of the pixel value of each pixel of the low-energy image and the high-energy image to the pixel value of the direct image is derived, and the ratio is further divided by the thickness of the artificial object to derive the artificial object attenuation coefficients µLa and µHa for various thicknesses of the artificial object. Since the thicknesses of the soft part and the bone part are also taken into consideration in the derivation of the artificial object attenuation coefficients µLa and µHa, the artificial object attenuation coefficients µLa and µHa are obtained in consideration of the beam hardening of the soft part and the bone part.

For example, in a case of deriving an artificial object attenuation coefficient µLa1(ts1,tb1,ta1) in a case of ts = ts1, tb = tb1, and ta = ta1, assuming that a pixel value of the acquired low-energy component image is CL1 and a pixel value of the direct image is CLd, the artificial object attenuation coefficient µLal(ts,tb1,ta1) is derived by Expression (13). $μLa 1 \left(ts1,tb1,ta1\right) = CL 1 / CLd$ (13)

Here, the thicknesses of the three types of substances included in the model are discrete. Therefore, for the three types of substances, the attenuation coefficients derived for various thicknesses can be interpolated to derive a relationship between the thickness and the attenuation coefficient of each component.

It should be noted that the soft part attenuation coefficients µLs and µHs derived as described above are represented by a one-dimensional look-up table that depends on only the thickness of the soft part. The bone part attenuation coefficients µLb and µHb are represented by a two-dimensional look-up table that depends on only the thicknesses of the soft part and the bone part. The artificial object attenuation coefficients µLa and µHa are represented by a three-dimensional look-up table that depends on the thicknesses of the soft part, the bone part, and the artificial object.

Here, as described above, there are a plurality of artificial objects having different materials, such as the metal, the silicone, and the contrast agent. Therefore, it is preferable to derive the artificial object attenuation coefficients µLa and µHa in advance in accordance with the type of the artificial object.

As described above, the component thickness derivation unit 24 derives the thickness ts of the soft part and the thickness tb of the bone part by solving Expression (11) and Expression (12) with the thickness ts of the soft part and the thickness tb of the bone part as variables. It should be noted that the thickness ts of the soft part and the thickness tb of the bone part, which are derived, are derived for each pixel of the first attenuation image CL and the second attenuation image CH, but, in the following description, (x,y) representing the pixel position will be omitted.

The component thickness derivation unit 24 first calculates the thickness tb of the bone part in a case in which the thickness ts of the soft part is set to zero by Expression (12). In a case in which ts = 0 and tb = tb0, CH = µHs(0, tb0, T - tb0 - 0) × 0 + µHb(0, tb0, T - tb0 - 0) × tb0 + µHa(0, tb0, T - tb0 - 0) × (T - tb0 - 0), and thus tb0 is calculated by Expression (14). In addition, the component thickness derivation unit 24 calculates the thickness ts0 of the soft part in a case in which the thickness tb of the bone part is set to zero, by Expression (12). In a case in which ts = ts0 and tb = 0, CH = µHs(ts0,0, T - 0 - ts0) × ts0 + µHb(ts0,0, T - 0 - ts0) × 0 + µHa(ts0,0,T - 0 - ts0) × (T - 0 - ts0), and thus ts0 is calculated by Expression (15). $tb 0 = CH / \left(μHb\left(0,tb0,T-tb0-0\right)\times tb0+μHa\left(0,tb0,T-tb0\right)\times \left(T-tb0\right)\right)$ (14) $ts 0 = CH / \left(μHs\left(ts0,0,T-0-ts0\right)\times ts0+μHa\left(ts0,0,T-ts0\right)\times \left(T-ts0\right)\right)$ (15)

Fig. 7 is a diagram showing a relationship between the attenuation amounts in accordance with the thickness of the bone part and the thickness of the soft part. In Fig. 7, an attenuation amount 33 indicates the attenuation amount CL which is the pixel value of the low-energy image and the attenuation amount CH which is the pixel value of the high-energy image which are derived by actually imaging the subject. Here, the attenuation amount of the low-energy image and the attenuation amount of the high-energy image are larger as the density of the composition is higher. Therefore, the composition in a case of tb = 0 and ts = ts0 has a lower density than the composition based on the actual thickness of the bone part and the actual thickness of the soft part. Therefore, in a case in which tb = 0 and ts = ts0, the pixel value, that is, the attenuation amount of the first attenuation image (here, a provisional first attenuation image CL') derived by Expression (11) is less than the pixel value of the first attenuation image CL derived from the actual thickness of the bone part and the actual thickness of the soft part as shown in an attenuation amount 34 of Fig. 7 (that is, CL > CL').

On the other hand, the composition in a case in which tb = tb0 and ts = 0 has a higher density than the composition based on the actual thickness of the bone part and the actual thickness of the soft part. Therefore, in a case in which tb = tb0 and ts = 0, the pixel value, that is, the attenuation amount of the provisional first attenuation image CL' derived by Expression (11) is greater than the pixel value of the first attenuation image CL derived from the actual thickness of the bone part and the actual thickness of the soft part as shown in an attenuation amount 35 of Fig. 7 (that is, CL < CL').

It should be noted that the pixel value, that is, the attenuation amount of the provisional first attenuation image CL' derived by Expression (11) by using the actual thickness of the bone part and the actual thickness of the soft part is the same as the pixel value of the first attenuation image CL as shown in an attenuation amount 36 of Fig. 7. By using this fact, the image derivation unit 25 derives the thickness tb of the bone part and the thickness ts of the soft part in the following manner.

### Step S1

First, in Expression (12), a provisional thickness tsk of the soft part is calculated by using the pixel value of the second attenuation image CH and the soft part attenuation coefficient µHs, the bone part attenuation coefficient µHb, and the artificial object attenuation coefficient µHa that are derived in each pixel. It should be noted that zero is used as an initial value of a provisional thickness tbk of the bone part.

### Step S2

Then, the provisional first attenuation image CL' is calculated by Expression (11) using the provisional thickness tsk of the soft part and the provisional thickness tbk of the bone part that are calculated, and the soft part attenuation coefficient µLs, the bone part attenuation coefficient µLb, and the artificial object attenuation coefficient µLa for the low-energy radiation. As the soft part attenuation coefficient µLs, the bone part attenuation coefficient µLb, and the artificial object attenuation coefficient µLa, the values corresponding to the provisional thickness tsk of the soft part, the provisional thickness tbk of the bone part, and the provisional thickness (that is, T - tsk - tbk) of the artificial object are used.

### Step S3

Next, the difference value ΔCL between the provisional first attenuation image CL' and the first attenuation image CL is calculated. The provisional thickness tbk of the bone part is updated on the assumption that the difference value ΔCL is the pixel value corresponding to the amount of the radiation attenuated by the bone.

### Step S4

Next, a provisional second attenuation image CH' is calculated by Expression (12) by using the updated provisional thickness tbk of the bone part and the provisional thickness tsk of the soft part.

### Step S5

Next, the difference value ΔCH between the provisional second attenuation image CH' and the second attenuation image CH is calculated. The provisional thickness tsk of the soft part is updated on the assumption that the difference value ΔCH is the pixel value corresponding to the amount of the radiation attenuated by the soft part.

Then, the thickness ts of the soft part and the thickness tb of the bone part are derived by repeating the processing of steps S1 to S5 until the absolute values of the difference values ΔCL and ΔCH are less than a predetermined threshold value. It should be noted that the thickness ts of the soft part and the thickness tb of the bone part may be derived by repeating the processing of steps S1 to S5 a predetermined number of times.

The image derivation unit 25 derives the first to third component images, that is, the soft part image Gs, the bone part image Gb, and the artificial object image Ga, based on the thickness ts of the soft part and the thickness tb of the bone part that are derived by the component thickness derivation unit 24 and the thickness ta of the artificial object derived by Expression (10). The soft part image Gs has a pixel value having a size in accordance with the thickness ts of the soft part. The bone part image Gb has a pixel value having a size in accordance with the thickness tb of the bone part. The artificial object image Ga has a pixel value having a size in accordance with the thickness ta of the artificial object (that is, T - ts - tb).

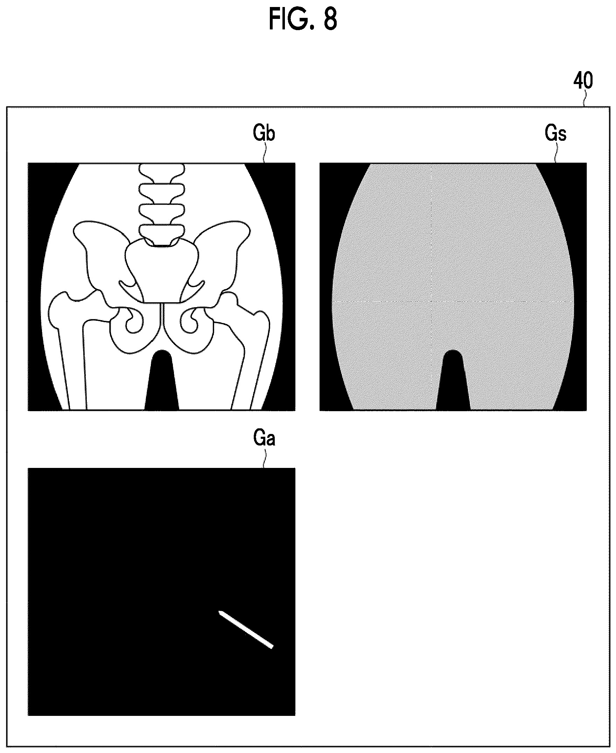

The display controller 26 displays the soft part image Gs, the bone part image Gb, and the artificial object image Ga that are derived. Fig. 8 is a diagram showing a display screen 40 of the soft part image Gs, the bone part image Gb, and the artificial object image Ga.

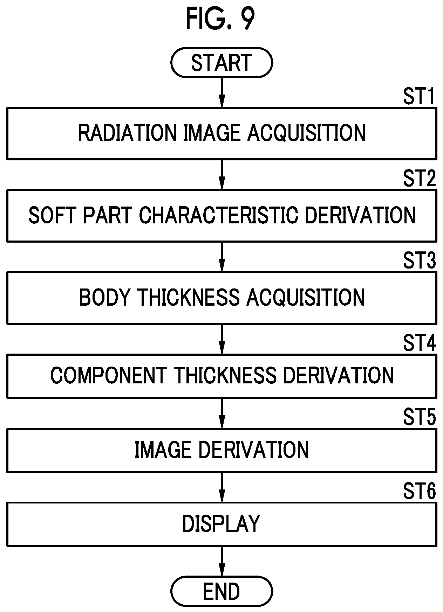

Hereinafter, the processing performed in the present embodiment will be described. Fig. 9 is a flowchart showing the processing performed in the present embodiment. The image acquisition unit 21 causes the imaging apparatus 1 to perform the energy subtraction imaging of the subject H to acquire the first and second radiation images G1 and G2 (radiation image acquisition: step ST1). Next, the characteristic derivation unit 22 derives the characteristics of the soft part component related to the attenuation of the radiation image in the soft region (step ST2). Next, the body thickness acquisition unit 23 acquires the body thickness T of the subject H (step ST3). It should be noted that the processing of step ST3 may be performed before the processing of step ST2, or may be performed in parallel with the processing of step ST2.

Then, the component thickness derivation unit 24 derives the thickness ts of the soft part, the thickness tb of the bone part, and the thickness ta of the artificial object by using the body thickness T, the characteristics of the soft part component, the first radiation image G1, and the second radiation image G2 (component thickness derivation: step ST4). Next, the image derivation unit 25 derives the soft part image Gs, the bone part image Gb, and the artificial object image Ga based on the thickness ts of the soft part, the thickness tb of the bone part, and the thickness ta of the artificial object (component image derivation: step ST5). Then, the display controller 26 displays the soft part image Gs, the bone part image Gb, and the artificial object image Ga (step ST6), and the processing ends.

As described above, in the present embodiment, the thickness ts of the soft part, the thickness tb of the bone part, and the thickness ta of the artificial object are derived by using the body thickness T, the characteristics of the soft part component, the first radiation image G1, and the second radiation image G2, and the soft part image Gs, the bone part image Gb, and the artificial object image Ga are derived based on the thickness ts of the soft part, the thickness tb of the bone part, and the thickness ta of the artificial object. Therefore, it is possible to acquire the soft part image Gs, the bone part image Gb, and the artificial object image Ga for the subject H only by acquiring two images having different energy distributions, which are the first and second radiation images G1 and G2. Therefore, the soft part component, the bone part component, and the artificial object component in the subject H can be accurately separated by using two images having different energy distributions, which are the first and second radiation images G1 and G2.

It should be noted that, in the embodiment described above, the images of three components of the soft part component, the bone part component, and the artificial object component in the subject H are derived by using the first and second radiation images G1 and G2, but the present disclosure is not limited to this. The first to (n - 1)th radiation images may be acquired by n - 1 types of radiation having different energy distributions, and the first to nth component images may be derived by using the body thickness and the first to (n - 1)th radiation images. For example, the soft part component and the bone part component in the subject H, and thicknesses of a first and second artificial object components having different compositions may be derived from the body thickness of the subject H and three radiation images having different energy distributions by using the three radiation images, to derive the soft part image, the bone part image, a first artificial object image, and a second artificial object image. Examples of the artificial objects having different compositions include metal such as the screw for fixing the fracture portion as the first artificial object and the stent disposed in the blood vessel, and the silicone disposed in the breast as the second artificial object. The artificial objects having different compositions also include as the contrast agents having different types.

Also, in the embodiment described above, the first and second radiation images G1 and G2 are acquired by the one-shot method, but the present disclosure is not limited to this. The first and second radiation images G1 and G2 may be acquired by a so-called two-shot method in which the imaging is performed twice by using only one radiation detector. In a case of the two-shot method, a position of the subject H included in the first radiation image G1 and the second radiation image G2 may shift due to a body movement of the subject H. Therefore, in the first radiation image G1 and the second radiation image G2, it is preferable to perform the processing according to the present embodiment after registration of the subject is performed.

In addition, in the embodiment described above, the radiation image acquired in the system that images the subject H using the first and second radiation detectors 5 and 6 is used, but it goes without saying that the technology of the present disclosure can be applied even in a case in which the first and second radiation images G1 and G2 are acquired using an accumulative phosphor sheet instead of the radiation detector. In this case, the first and second radiation images G1 and G2 need only be acquired by stacking two accumulative phosphor sheets, emitting the radiation transmitted through the subject H, accumulating and recording radiation image information of the subject H in each of the accumulative phosphor sheets, and photoelectrically reading the radiation image information from each of the accumulative phosphor sheets. It should be noted that the two-shot method may also be used in a case in which the first and second radiation images G1 and G2 are acquired by using the accumulative phosphor sheet.

In addition, the radiation in the embodiment described above is not particularly limited, and α-rays or γ-rays can be used in addition to X-rays.

In addition, in the embodiment described above, various processors shown below can be used as the hardware structure of processing units that execute various types of processing, such as the image acquisition unit 21, the characteristic derivation unit 22, the body thickness acquisition unit 23, the component thickness derivation unit 24, the image derivation unit 25, and the display controller 26. As described above, the various processors include, in addition to the CPU that is a general-purpose processor which executes software (program) and functions as various processing units, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application specific integrated circuit (ASIC).

One processing unit may be configured by one of these various processors, or may be configured by a combination of two or more processors of the same type or different types (for example, a combination of a plurality of FPGAs or a combination of the CPU and the FPGA). In addition, a plurality of the processing units may be configured by one processor.

As an example of configuring the plurality of processing units by one processor, first, as represented by a computer of a client, a server, and the like there is an aspect in which one processor is configured by a combination of one or more CPUs and software and this processor functions as a plurality of processing units. Second, as represented by a system on chip (SoC) or the like, there is an aspect of using a processor that implements the function of the entire system including the plurality of processing units by one integrated circuit (IC) chip. In this way, as the hardware structure, the various processing units are configured by using one or more of the various processors described above.

Further, as the hardware structures of these various processors, more specifically, it is possible to use an electrical circuit (circuitry) in which circuit elements, such as semiconductor elements, are combined.

Hereinafter, the supplementary notes of the present disclosure will be described.

### Supplementary Note 1

A radiation image processing device comprising: at least one processor, in which the processor is configured to: acquire first to (n - 1)th (n ≥ 3) radiation images acquired by imaging a subject, which includes a first component consisting of a plurality of compositions and second to nth components each consisting of a single composition, with n - 1 types of radiation having different energy distributions; derive a characteristic of the first component in at least a region of the subject in the first to (n - 1)th radiation images; acquire a body thickness of the subject; derive thicknesses of the first to nth components by using the body thickness, the characteristic of the first component, and the first to (n - 1)th radiation images; and derive first to nth component images in which the first to nth components are enhanced, respectively, based on the thicknesses of the first to nth components.

### Supplementary Note 2

The radiation image processing device according to supplementary note 1, in which the processor is configured to acquire the body thickness by deriving the body thickness of the subject based on at least one of the first to (n - 1)th radiation images.

### Supplementary Note 3

The radiation image processing device according to supplementary note 1 or 2, in which the processor is configured to derive an attenuation coefficient of the first component as the characteristic of the first component.

### Supplementary Note 4

The radiation image processing device according to supplementary note 3, in which the processor is configured to derive the attenuation coefficient of the first component as the characteristic of the first component based on information related to an attenuation coefficient of the radiation of each of the plurality of compositions included in the first component and a thickness of each of the plurality of compositions.

### Supplementary Note 5

The radiation image processing device according to any one of supplementary notes 1 to 4, in which the processor is configured to: acquire a first radiation image and a second radiation image that are acquired by imaging the subject with two types of radiation having different energy components; derive the characteristic of the first component in at least a region of the subject in the first radiation image or the second radiation image; derive the body thickness of the subject based on at least one of the first radiation image or the second radiation image; derive thicknesses of the first component, a second component, and a third component of the subject by using the body thickness, the characteristic of the first component, the first radiation image, and the second radiation image; and derive a first component image, a second component image, and a third component image in which the first component, the second component, and the third component are enhanced, respectively, based on the thicknesses of the first component, the second component, and the third component.

### Supplementary Note 6

The radiation image processing device according to any one of supplementary notes 1 to 5, in which the first component, the second component, and the third component are a soft part and a bone part of the subject and an artificial object in the subject, respectively.

### Supplementary Note 7

The radiation image processing device according to supplementary note 6, in which the compositions of the first component are fat and muscle.

### Supplementary Note 8

A radiation image processing method executed by a computer, the radiation image processing method comprising: acquiring first to (n - 1)th (n ≥ 3) radiation images acquired by imaging a subject, which includes a first component consisting of a plurality of compositions and second to nth components each consisting of a single composition, with n - 1 types of radiation having different energy distributions; deriving a characteristic of the first component in at least a region of the subject in the first to (n - 1)th radiation images; acquiring a body thickness of the subject; deriving thicknesses of the first to nth components by using the body thickness, the characteristic of the first component, and the first to (n - 1)th radiation images; and deriving first to nth component images in which the first to nth components are enhanced, respectively, based on the thicknesses of the first to nth components.

### Supplementary Note 9

A radiation image processing program causing a computer to execute: a procedure of acquiring first to (n - 1)th (n ≥ 3) radiation images acquired by imaging a subject, which includes a first component consisting of a plurality of compositions and second to nth components each consisting of a single composition, with n - 1 types of radiation having different energy distributions; a procedure of deriving a characteristic of the first component in at least a region of the subject in the first to (n - 1)th radiation images; a procedure of acquiring a body thickness of the subject; a procedure of deriving thicknesses of the first to nth components by using the body thickness, the characteristic of the first component, and the first to (n - 1)th radiation images; and a procedure of deriving first to nth component images in which the first to nth components are enhanced, respectively, based on the thicknesses of the first to nth components.

### Explanation of References

1: imaging apparatus
3: radiation source
5, 6: radiation detector
7: radiation energy conversion filter
10: image processing device
11: CPU
12: radiation image processing program
13: storage
14: display
15: input device
16: memory
17: network I/F
18: bus
21: image acquisition unit
22: characteristic derivation unit
23: body thickness acquisition unit
24: component thickness derivation unit
25: image derivation unit
26: display controller
33 to 36: attenuation amount
40: display screen
Ga: artificial object image
Gb: bone part image
Gs: soft part image
H: subject

## Claims

1. A radiation image processing device comprising:
at least one processor (11),
wherein the processor is configured to:
acquire first to (n-1)th, wherein n ≥ 3, radiation images acquired by imaging a subject (H), which includes a first component consisting of a plurality of compositions and second to nth components each consisting of a single composition, with n - 1 types of radiation having different energy distributions;
derive a characteristic of the first component in at least a region of the subject in the first to (n - 1)th radiation images;
acquire a body thickness of the subject;
**characterised in that** the processor is further configured to
derive thicknesses of the first to nth components by using the body thickness, the characteristic of the first component, and the first to (n - 1)th radiation images; and
derive first to nth component images in which the first to nth components are enhanced, respectively, based on the thicknesses of the first to nth components.

2. The radiation image processing device according to claim 1,
wherein the processor (11) is configured to acquire the body thickness by deriving the body thickness of the subject based on at least one of the first to (n - 1)th radiation images.

3. The radiation image processing device according to claim 1 or 2,
wherein the processor (11) is configured to derive an attenuation coefficient of the first component as the characteristic of the first component.

4. The radiation image processing device according to claim 3,
wherein the processor (11) is configured to derive the attenuation coefficient of the first component as the characteristic of the first component based on information related to an attenuation coefficient of the radiation of each of the plurality of compositions included in the first component and a thickness of each of the plurality of compositions.

5. The radiation image processing device according to any one of claims 1 to 4,
wherein the processor (11) is configured to:
acquire a first radiation image and a second radiation image that are acquired by imaging the subject with two types of radiation having different energy components;
derive the characteristic of the first component in at least a region of the subject in the first radiation image or the second radiation image;
derive the body thickness of the subject based on at least one of the first radiation image or the second radiation image;
derive thicknesses of the first component, a second component, and a third component of the subject by using the body thickness, the characteristic of the first component, the first radiation image, and the second radiation image; and
derive a first component image, a second component image, and a third component image in which the first component, the second component, and the third component are enhanced, respectively, based on the thicknesses of the first component, the second component, and the third component.

6. The radiation image processing device according to any one of claims 1 to 5,
wherein the first component, the second component, and the third component are a soft part and a bone part of the subject and an artificial object in the subject, respectively.

7. The radiation image processing device according to claim 6,
wherein the compositions of the first component are fat and muscle.

8. A radiation image processing method executed by a computer, the radiation image processing method comprising:
acquiring first to (n-1)th, wherein n ≥ 3, radiation images acquired by imaging a subject, which includes a first component consisting of a plurality of compositions and second to nth components each consisting of a single composition, with n - 1 types of radiation having different energy distributions;
deriving a characteristic of the first component in at least a region of the subject in the first to (n - 1)th radiation images;
acquiring a body thickness of the subject;
**characterised in**
deriving thicknesses of the first to nth components by using the body thickness, the characteristic of the first component, and the first to (n - 1)th radiation images; and
deriving first to nth component images in which the first to nth components are enhanced, respectively, based on the thicknesses of the first to nth components.

9. A computer-readable storage medium that stores a radiation image processing program causing a computer to execute:
a procedure of acquiring first to (n - 1)th (n ≥ 3) radiation images acquired by imaging a subject, which includes a first component consisting of a plurality of compositions and second to nth components each consisting of a single composition, with n - 1 types of radiation having different energy distributions;
a procedure of deriving a characteristic of the first component in at least a region of the subject in the first to (n - 1)th radiation images;
a procedure of acquiring a body thickness of the subject;
a procedure of deriving thicknesses of the first to nth components by using the body thickness, the characteristic of the first component, and the first to (n - 1)th radiation images; and
a procedure of deriving first to nth component images in which the first to nth components are enhanced, respectively, based on the thicknesses of the first to nth components.

## Patentansprüche

1. Strahlungsbild-Verarbeitungsvorrichtung, umfassend:
mindestens einen Prozessor (11),
wobei der Prozessor so konfiguriert ist, dass er:
ein erstes bis (n - 1)-tes, wobei n ≥ 3 ist, Strahlungsbild erfasst, die durch Abbilden einer Untersuchungsperson (H), die eine erste Komponente, die aus mehreren Zusammensetzungen besteht, und eine zweite bis n-te Komponente, die jeweils aus einer einzigen Zusammensetzung bestehen, enthält, mit n - 1 Typen von Strahlung, die unterschiedliche Energieverteilungen aufweisen, erfasst werden;
eine Eigenschaft der ersten Komponente in mindestens einem Bereich der Untersuchungsperson in dem ersten bis (n - 1)-ten Strahlungsbild ableitet;
eine Körperdicke der Untersuchungsperson erfasst;
**dadurch gekennzeichnet, dass** der Prozessor ferner so konfiguriert ist, dass er:
Dicken der ersten bis n-ten Komponente durch Verwenden der Körperdicke, der Eigenschaft der ersten Komponente und des ersten bis (n - 1)-ten Strahlungsbildes ableitet; und
ein erstes bis n-tes Komponentenbild, in denen die erste bis n-te Komponente entsprechend verbessert sind, auf der Grundlage der Dicken der ersten bis n-ten Komponente ableitet.

2. Strahlungsbild-Verarbeitungsvorrichtung nach Anspruch 1,
wobei der Prozessor (11) so konfiguriert ist, dass er die Körperdicke durch Ableiten der Körperdicke der Untersuchungsperson auf der Grundlage mindestens eines von dem ersten bis (n - 1)-ten Strahlungsbild erfasst.

3. Strahlungsbild-Verarbeitungsvorrichtung nach Anspruch 1 oder 2,
wobei der Prozessor (11) so konfiguriert ist, dass er einen Dämpfungskoeffizienten der ersten Komponente als die Eigenschaft der ersten Komponente ableitet.

4. Strahlungsbild-Verarbeitungsvorrichtung nach Anspruch 3,
wobei der Prozessor (11) so konfiguriert ist, dass er den Dämpfungskoeffizienten der ersten Komponente als die Eigenschaft der ersten Komponente auf der Grundlage von Informationen, die sich auf einen Dämpfungskoeffizienten der Strahlung jeder der mehreren Zusammensetzungen, die bei der ersten Komponente enthalten sind, und eine Dicke jeder der mehreren Zusammensetzungen beziehen, ableitet.

5. Strahlungsbild-Verarbeitungsvorrichtung nach einem der Ansprüche 1 bis 4,
wobei der Prozessor (11) so konfiguriert ist, dass er:
ein erstes Strahlungsbild und ein zweites Strahlungsbild, die durch Abbilden der Untersuchungsperson mit zwei Typen von Strahlung, die unterschiedliche Energiekomponenten aufweisen, erfasst werden, erfasst;
die Eigenschaft der ersten Komponente in mindestens einem Bereich der Untersuchungsperson in dem ersten Strahlungsbild oder dem zweiten Strahlungsbild ableitet;
die Körperdicke der Untersuchungsperson auf der Grundlage mindestens eines von dem ersten Strahlungsbild und dem zweiten Strahlungsbild ableitet;
Dicken der ersten Komponente, einer zweiten Komponente und einer dritten Komponente der Untersuchungsperson durch Verwenden der Körperdicke, der Eigenschaft der ersten Komponente, des ersten Strahlungsbildes und des zweiten Strahlungsbildes ableitet; und
ein erstes Komponentenbild, ein zweites Komponentenbild und ein drittes Komponentenbild, in denen die erste Komponente, die zweite Komponente und die dritte Komponente entsprechend verbessert sind, auf der Grundlage der Dicken der ersten Komponente, der zweiten Komponente und der dritten Komponente ableitet.

6. Strahlungsbild-Verarbeitungsvorrichtung nach einem der Ansprüche 1 bis 5,
wobei die erste Komponente, die zweite Komponente und die dritte Komponente entsprechend ein weicher Teil und ein Knochenteil der Untersuchungsperson und ein künstliches Objekt in der Untersuchungsperson sind.

7. Strahlungsbild-Verarbeitungsvorrichtung nach Anspruch 6,
wobei die Zusammensetzungen der ersten Komponente Fett und Muskel sind.

8. Strahlungsbild-Verarbeitungsverfahren, das von einem Computer ausgeführt wird,
wobei das Strahlungsbild-Verarbeitungsverfahren umfasst:
Erfassen eines ersten bis (n - 1)-ten, wobei n ≥ 3 ist, Strahlungsbildes, die durch Abbilden einer Untersuchungsperson, die eine erste Komponente, die aus mehreren Zusammensetzungen besteht, und eine zweite bis n-te Komponente, die jeweils aus einer einzigen Zusammensetzung bestehen, enthält, mit n - 1 Typen von Strahlung, die unterschiedliche Energieverteilungen aufweisen, erfasst werden;
Ableiten einer Eigenschaft der ersten Komponente in mindestens einem Bereich der Untersuchungsperson in dem ersten bis (n - 1)-ten Strahlungsbild;
Erfassen einer Körperdicke der Untersuchungsperson;
**gekennzeichnet durch**
Ableiten von Dicken der ersten bis n-ten Komponente durch Verwenden der Körperdicke, der Eigenschaft der ersten Komponente und des ersten bis (n - 1)-ten Strahlungsbildes; und
Ableiten von erstem bis n-tem Komponentenbild, in denen die erste bis n-te Komponente entsprechend verbessert sind, auf der Grundlage der Dicken der ersten bis n-ten Komponente.

9. Computerlesbares Speichermedium, das ein Strahlungsbild-Verarbeitungsprogramm speichert, das einen Computer veranlasst, auszuführen:
ein Verfahren des Erfassens eines ersten bis (n - 1)-ten (n ≥ 3) Strahlungsbildes, die durch Abbilden einer Untersuchungsperson, die eine erste Komponente, die aus mehreren Zusammensetzungen besteht, und eine zweite bis n-te Komponente, die jeweils aus einer einzigen Zusammensetzung bestehen, enthält, mit n - 1 Typen von Strahlung, die unterschiedliche Energieverteilungen aufweisen, erfasst werden;
ein Verfahren des Ableitens einer Eigenschaft der ersten Komponente in mindestens einem Bereich der Untersuchungsperson in dem ersten bis (n - 1)-ten Strahlungsbild;
ein Verfahren des Erfassens einer Körperdicke der Untersuchungsperson;
ein Verfahren des Ableitens von Dicken der ersten bis n-ten Komponente durch Verwenden der Körperdicke, der Eigenschaft der ersten Komponente und des ersten bis (n - 1)-ten Strahlungsbildes; und
ein Verfahren des Ableitens von erstem bis n-tem Komponentenbild, in denen die erste bis n-te Komponente entsprechend verbessert sind, auf der Grundlage der Dicken der ersten bis n-ten Komponente.

## Revendications

1. Dispositif de traitement d'images de rayonnement comprenant :
au moins un processeur (11),
dans lequel le processeur est configuré pour :
acquérir des première à (n - 1)-ièmes images de rayonnement, où n ≥ 3, acquises en imageant un sujet (H), qui inclut un premier composant constitué d'une pluralité de compositions et des deuxièmes à n-ièmes composants constitués chacun d'une composition unique, au moyen de n - 1 types de rayonnement ayant des distributions d'énergie différentes ;
dériver une caractéristique du premier composant dans au moins une région du sujet dans les première à (n - 1)-ièmes images de rayonnement ;
acquérir une épaisseur corporelle du sujet ;
**caractérisé en ce que** le processeur est en outre configuré pour
dériver des épaisseurs des premier à n-ièmes composants en utilisant l'épaisseur corporelle, la caractéristique du premier composant et les première à (n - 1)-ièmes images de rayonnement ; et
dériver des première à n-ièmes images de composants dans lesquelles les premier à n-ièmes composants sont respectivement accentués, sur la base des épaisseurs des premier à n-ièmes composants.

2. Dispositif de traitement d'images de rayonnement selon la revendication 1,
dans lequel le processeur (11) est configuré pour acquérir l'épaisseur corporelle en dérivant l'épaisseur corporelle du sujet sur la base d'au moins l'une des première à (n - 1)-ièmes images de rayonnement.

3. Dispositif de traitement d'images de rayonnement selon la revendication 1 ou la revendication 2,
dans lequel le processeur (11) est configuré pour dériver un coefficient d'atténuation du premier composant comme caractéristique du premier composant.

4. Dispositif de traitement d'images de rayonnement selon la revendication 3,
dans lequel le processeur (11) est configuré pour dériver le coefficient d'atténuation du premier composant comme caractéristique du premier composant sur la base d'informations relatives à un coefficient d'atténuation du rayonnement de chacune de la pluralité de compositions incluses dans le premier composant et à une épaisseur de chacune de la pluralité de compositions.

5. Dispositif de traitement d'images de rayonnement selon l'une quelconque des revendications 1 à 4,
dans lequel le processeur (11) est configuré pour :
acquérir une première image de rayonnement et une deuxième image de rayonnement qui sont acquises en imageant le sujet au moyen de deux types de rayonnement ayant des composants d'énergie différentes ;
dériver la caractéristique du premier composant dans au moins une région du sujet dans la première image de rayonnement ou la deuxième image de rayonnement ;
dériver l'épaisseur corporelle du sujet sur la base d'au moins l'une de la première image de rayonnement ou de la deuxième image de rayonnement ;
dériver des épaisseurs du premier composant, d'un deuxième composant et d'un troisième composant du sujet en utilisant l'épaisseur corporelle, la caractéristique du premier composant, la première image de rayonnement et la deuxième image de rayonnement ; et
dériver une première image de composant, une deuxième image de composant et une troisième image de composant dans lesquelles le premier composant, le deuxième composant et le troisième composant sont respectivement accentués, sur la base des épaisseurs du premier composant, du deuxième composant et du troisième composant.

6. Dispositif de traitement d'images de rayonnement selon l'une quelconque des revendications 1 à 5,
dans lequel le premier composant, le deuxième composant et le troisième composant sont respectivement une partie molle et une partie osseuse du sujet et un objet artificiel dans le sujet.

7. Dispositif de traitement d'images de rayonnement selon la revendication 6,
dans lequel les compositions du premier composant sont de la graisse et du muscle.

8. Procédé de traitement d'images de rayonnement exécuté par un ordinateur, le procédé de traitement d'images de rayonnement comprenant :
acquérir des première à (n - 1)-ièmes images de rayonnement, où n ≥ 3, acquises en imageant un sujet, qui inclut un premier composant constitué d'une pluralité de compositions et des deuxièmes à n-ièmes composants constitués chacun d'une composition unique, au moyen de n - 1 types de rayonnement ayant des distributions d'énergie différentes ;
dériver une caractéristique du premier composant dans au moins une région du sujet dans les première à (n - 1)-ièmes images de rayonnement ;
acquérir une épaisseur corporelle du sujet ;
**caractérisé par**
dériver des épaisseurs des premier à n-ièmes composants en utilisant l'épaisseur corporelle, la caractéristique du premier composant et les première à (n - 1)-ièmes images de rayonnement ; et
dériver des première à n-ièmes images de composants dans lesquelles les premier à n-ièmes composants sont respectivement accentués, sur la base des épaisseurs des premier à n-ièmes composants.

9. Support de stockage lisible par ordinateur qui stocke un programme de traitement d'images de rayonnement amenant un ordinateur à exécuter :
une procédure consistant à acquérir des première à (n - 1)-ièmes images de rayonnement (n ≥ 3) acquises en imageant un sujet, qui inclut un premier composant constitué d'une pluralité de compositions et des deuxièmes à n-ièmes composants constitués chacun d'une composition unique, au moyen de n - 1 types de rayonnement ayant des distributions d'énergie différentes ;
une procédure consistant à dériver une caractéristique du premier composant dans au moins une région du sujet dans les première à (n - 1)-ièmes images de rayonnement ;
une procédure consistant à acquérir une épaisseur corporelle du sujet ;
une procédure consistant à dériver des épaisseurs des premier à n-ièmes composants en utilisant l'épaisseur corporelle, la caractéristique du premier composant et les première à (n - 1)-ièmes images de rayonnement ; et
une procédure consistant à dériver des première à n-ièmes images de composants dans lesquelles les premier à n-ièmes composants sont respectivement accentués, sur la base des épaisseurs des premier à n-ièmes composants.
